# EUROPÄISCHE PATENTSCHRIFT

(11) **EP 3 515 538 B1**
(45) Veröffentlichungstag und Bekanntmachung des Hinweises auf die Patenterteilung: **15.11.2023**
(21) Anmeldenummer: 17764753.4
(22) Anmeldetag: 06.09.2017
(51) Int. Cl.: A61M 5/32, A61M 5/20, A61M 5/50

(54) **NADELSCHUTZABZIEHKAPPE FUR INJEKTIONSVORRICHTUNG**
DETACHABLE NEEDLE PROTECTION CAP FOR INJECTION DEVICES
CAPUCHON À TIRER DE PROTECTION D'AIGUILLE POUR DISPOSITIF D'INJECTION

(30) Priorität: 21.09.2016 CH 12242016
(43) Veröffentlichungstag der Anmeldung: 31.07.2019
(73) Patentinhaber: Ypsomed AG, 3401 Burgdorf (CH)
(72) Erfinder: TSCHIRREN, Markus, 3400 Burgdorf (CH); MOSER, Ulrich, 3412 Heimiswil (CH); FIECHTER, Marc, 3510 Konolfingen (CH); KLÖTZLI, Urs, Thornbury, VIC 3071 (AU); GROETZBACH, Felix, 4563 Gerlafingen (CH)
(74) Vertreter: Meier Obertüfer, Jürg
(86) Internationale Anmeldenummer: PCT/CH2017/000081
(87) Internationale Veröffentlichungsnummer: WO 2018/053657

(56) Entgegenhaltungen:
- EP-A1- 2 745 866
- WO-A1-2014/111370
- GB-A- 2 100 380
- US-A1- 2014 364 804

## Beschreibung

### Technisches Gebiet

Die Erfindung betrifft Nadelschutzabziehkappen für Injektionsvorrichtungen, insbesondere für Injektionspens und Autoinjektoren, in welchen vorbefüllte Spritzen als Produktbehälter zum Einsatz gelangen, und mit welchen ein im Produktbehälter enthaltenes fluides Produkt ausschüttbar ist. Bei dem fluiden Produkt handelt es sich insbesondere um ein Medikament. Im Besonderen betrifft die Erfindung Nadelschutzabziehkappen für Injektionsvorrichtungen, welche kostengünstig an die Bedürfnisse verschiedener medizinischer Indikationen anpassbar ist.

### Hintergrund

Eine Injektionsvorrichtung, wie ein Injektionspen, ein Autopen oder ein Autoinjektor, kann grundsätzlich für die Verabreichung von verschiedensten Medikamenten geeignet sein, sofern das Medikament eine Konsistenz aufweist, welche mit dem Injektionsgerät ausschüttbar ist. Unterschiede ergeben sich jedoch indirekt durch die medizinischen Indikationen, für welche die verschiedenen Medikamente verwendet werden. So können sich aufgrund der Indikation verschiedene Ansprüche an Grösse, Form, Oberfläche und mechanische Eigenschaften des Gehäuses des Injektionsgerätes stellen. So kann zum Beispiel bei Krankheiten wie multipler Sklerose (MS) die Beweglichkeit des Patienten eingeschränkt sein, so dass an eine Injektionsvorrichtung, welche verwendet wird, um Patienten mit MS zu behandeln, besondere Anforderungen an die Form des Gerätes gestellt werden, damit die Patienten das Injektionsgerät überhaupt handhaben können. So kann es bei medizinischen Indikationen, welche die Handhabung von Injektionsgeräten durch Kinder oder kleinwüchsige Menschen betreffen, erforderlich sein, dass Injektionsvorrichtungen möglichst schlank und klein ausgestaltet sind.

Bei Injektionsgeräten, bei welchen eine vorbefüllte Spritze als Produktbehältnis eingesetzt wird, wird häufig eine sogenannte Nadelschutzabziehkappe vewendet, um den auf der Spritze vorhandenen Nadelschutz vor einem Injektionsvorgang entfernen zu können. Mit Hilfe der Nadelschutzabziehkappe kann die benutzende Person den Nadelschutz manuell von der Spritze entfernen. Es wird unmittelbar offensichtlich, dass die Nadelschutzabziehkappe in Form und Gestaltung ebenfalls der Anwendung (wie oben beschrieben) angepasst werden muss.

Der Begriff "Medikament" umfasst hier jede fliessfähige medizinische Formulierung, welche geeignet ist zur kontrollierten Verabreichung durch ein Mittel, wie z. B. eine Kanüle oder Hohlnadel, hindurch, beispielsweise umfassend eine Flüssigkeit, eine Lösung, ein Gel, eine Emulsion oder eine feine Suspension, welche(s) einen oder mehrere medizinische Wirkstoffe enthält. "Medikament" kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form aber auch Polysaccharide, Vakzine, DNS oder RNS oder Oligonukleotide, Antikörper oder Teile von Antikörpern sowie geeignete Basis-, Hilfs- und Trägerstoffe.

Aus der WO15110532 A1 ist ein Injektionsgerät bekannt, welches eine mehrteilige Nadelschutzabziehkappe umfasst. Dabei umfasst die Nadelschutzabziehkappe eine Kappe 50 und einen Einsatz 90, welche über eine einfache Schnappverbindung miteinander verbunden sind. Für die Kappe 50 ist eine bestimmte Variante vorgesehen, welche einteilig ausgeführt ist.

Die US 2014/364804 A1 offenbart eine Manipulationssicherung für eine Nadelhalterung, um ein unbefugtes Entfernen der Nadeleinheit von einem Injektionsgerät zu verhindern. Die Manipulationssicherung umfasst zwei halbschalenförmige Teile, welche je Schnapparme aufweisen, die in das gegenüberliegende Teil einschnappen, wenn die beiden Teile um den Nadelbefestigungsbereich am Injektionsgerät gelegt werden. Wenn die Teile weiter gegeneinander zubewegt werden, werden die Schnapparme in eine Sicherungsstellung bewegt, so dass sie die beiden Teile fest zueinander am Nadelbefestigungsbereich gehalten sind.

Die EP 2 745 866 B1 offenbart einen Autoinjektor mit einer Auslösesicherung, um eine ungewollte Ausschüttung eines Medikaments zu verhindern. Hierzu weist der Autoinjektor eine Abdeckkappe mit einem Schnapphaken auf, der in eine Lücke zwischen einem Spritzenkörper und einem proximalen Ende einer Nadelabdeckkappe eingreift. Wenn die Abdeckkappe von dem Autoinjektor entfernt wird, hakt der Schnapper in das proximale Ende der Nadelabdeckkappe ein, wodurch die Nadelabdeckkappe von einem Produktbehälter gelöst und zusammen mit der Abdeckkappe von dem Autoinjektor entfernt wird.

Die WO 2015/110532 A1 offenbart einen Autoinjektor mit einer abnehmbaren Kappe, welche eine Nadelschutzhülse mittels einem Einlegeteil umschliesst. Die Kappe weist im Inneren axiale Führungen auf, in denen bei der Montage Schnapper des Einlegeteils axial geführt werden bis diese in Öffnungen in der Kappe einschnappen, so dass das Einlegeteil mit der Kappe verbunden ist. Wenn vor Gebrauch des Autoinjektors die Kappe abgezogen wird, wird mittels der Schnapper das Einlegeteil ebenfalls vom Autoinjektor abgezogen.

Die WO 2014/146209 beschreibt einen Autoinjektor mit einem Kappenabzugselement, welches vor Benutzung des Autoinjektors in distaler Richtung abgezogen werden muss. Das Kappenabzugselement weist Schnapphacken auf, welche hinter einem hinteren Rand einer Nadelschutzkappe eingreifen. Auf der radial äusseren Seite der Schnapphaken vorgesehene an diesen anliegende Rippen des Gehäuses verhindert ein Ausweichen dieser Schnapphaken, so dass durch ein Ziehen an dem Kappenabzugselement die Nadelschutzkappe in distaler Richtung abgezogen werden kann.

Für Hersteller von Injektionsvorrichtungen, Medikamentenhersteller, Krankenversicher und zu versorgende Personen ist es ein Bedürfnis, Injektionsvorrichtungen zur Hand zu haben, welche möglichst günstig, jedoch sicher und wirksam sind. Entsprechend wäre es wünschbar, eine Nadelschutzabziehkappe für eine Injektionsvorrichtung zu haben, welche ein gleichbleibendes, bewährtes, sicheres und vorgegebenes technisches Design mit einem gleichbleibenden Gehäuse aufweist, wobei über zusätzliche Anbauteile, welche fest und unlösbar mit dem Gehäuse der Injektionsvorrichtung wie auch der Nadelschutzabziehkappe verbunden werden, Form, Haptik, Oberflächentopographie und Ergonomie der Injektionsvorrichtung noch während der Produktion der Vorrichtung angepasst werden können, womit den Bedürfnissen der medizinischen Indikation auf einfache und günstige Weise Rechnung getragen werden kann.

Es ist eine Aufgabe der Erfindung Nadelschutzabziehkappen für Injektionsvorrichtungen bereitzustellen, welche kostengünstig an die Bedürfnisse verschiedener medizinischer Indikationen anpassbar ist.

### Allgemeine Beschreibung

Die Aufgabe wird erfindungsgemäss durch die Vorrichtung nach dem unabhängigen Anspruch 1 gelöst, vorteilhafte Weiterentwicklungen ergeben sich aus den abhängigen Ansprüchen, der Beschreibung und den Figuren.

In der folgenden Beschreibung werden die Begriffe distal und proximal in Bezug auf Positions- und Richtungsangaben verwendet. Hierbei meint zum Beispiel distales Ende der Injektionsvorrichtung dasjenige Ende der Injektionsvorrichtung, an welchem die Injektionsnadel angebracht ist. Die distale Richtung bezeichnet sodann die Richtung, in welche die Injektionsnadelspitze weist. Proximal bezeichnet also dann das dem distalen Ende entgegengesetzte Ende, resp. die Richtung welche der distalen Richtung entgegen gesetzt ist.

Die Funktion der Nadelschutzabziehkappe wird im Folgenden kurz am Beispiel eines Autoinjektors in Stiftform kurz erklärt. Es könnten jedoch auch andere Injektionsgeräte mit einer erfindungsgemässen Nadelschutzabziehkappe verwendet werden, wenn in diesen eine vorbefüllte Spritze als Produktbehältnis zum Einsatz gelangt. Insbesondere kann es sich bei den Injektionsgeräten auch um Injektionspens oder Autopens handeln.

In Autoinjektoren kommen typischerweise vorbefüllte Spritzen als Medikamentenbehälter zum Einsatz. Am distalen Ende der Spritze ist die Injektionsnadel fest angeordnet. Die Injektionsnadel wird dabei vor der Verwendung durch einen Nadelschutz in Form einer Gummikappe (ev. mit einem versteiften Stützgerüst) geschützt, wobei die Gummikappe auch eine Sterilbarriere bildet. Bei der Montage des Autoinjektors wird die vorbefüllte Spritze in den Autoinjektor eingeschoben und darin fixiert. Den distalen Abschluss des Autoinjektors bildet die Nadelschutzabziehkappe, welche es erlaubt, vor dem Gebrauch des Autoinjektors den Nadelschutz von der vorbefüllten Spritze zu entfernen, ohne dass für die benutzende Person das Risiko besteht sich an der Injektionsnadel zu stechen.

In einem Aspekt der Erfindung weist die Nadelschutzabziehkappe ein Gehäuse auf. Das Gehäuse definiert dabei zusammen mit dem Autoinjektor eine Längsachse, und senkrecht zur Längsachse werden Radien definiert. Zum Abziehen des Nadelschutzes von der vorbefüllten Spritze sind am Gehäuse mindestens zwei flexible Arme angeordnet, welche in die proximale Richtung schauen und deren proximale Enden über hakenartige Strukturen verfügen, welche, wenn die Nadelschutzabziehkappe auf den Autoinjektor aufgesetzt ist, hinter das proximal Ende des Nadelschutzes radial nach innen ragen. Wird die Nadelschutzabziehkappe in die distale Richtung nun vom Autoinjektor abgezogen, so greifen die hakenartigen Strukturen in den den Nadelschutz ein und ziehen diesen von der vorbefüllten Spritze ab.

Das Gehäuse der Nadelschutzkappe hat grundsätzlich eine etwa zylindrische Form, wobei das Gehäuse Bereiche mit unterschiedlichen Durchmessern aufweisen kann. Auf der äusseren Oberfläche des Gehäuses der Nadelschutzabziehkappe sind zusätzliche Elemente angeordnet, welche das Anbringen und Fixieren von zusätzlichen Teilen ermöglicht.

In einem Aspekt der Erfindung umfasst die Nadelschutzabziehkappe weiter zumindest zwei Schalenteile, welche auf der Aussenseite des Gehäuses angebracht werden können. Die Schalenteile umfassen dabei Komplementärelemente, insbesondere Befestigungs- und Führungselemente, die es erlauben, wenn in Eingriff mit den Elementen auf der Aussenseite des Gehäuses gebracht, die Schalenteile am Gehäuse zu fixieren. Weiter umfassen die Schalenteile Blockierelemente, welche verhindern können, dass die Schalenteile nach dem Anbringen am Gehäuse wieder gelöst werden können. Erfindungsgemäss blockieren die Blockierelemente eines Schalenteils das Lösen der Komplementärelemente eines anderen Schalenteils und umgekehrt, was den Vorteil hat, dass wenn alle der mindestens zwei Schalenteile am Gehäuse fixiert sind, ein zerstörungsfreies Lösen der Schalenteile verunmöglicht wird (ohne das Teile der Nadelschutzabziehkappe beschädigt werden).

Die Positionierung der Komplementärelemente und der Blockierlemente an den Schalenteilen ergibt sich aus der Anordnung der Elemente auf der äusseren Oberfläche des Gehäuses.

Die mindestens zwei Schalenteile ermöglichen es, die äussere Form der Nadelschutzabziehkappe an spezifische Bedürfnisse anzupassen, ohne dabei Änderungen am Gehäuse und der Funktion der Nadelschutzabziehkappe vornehmen zu müssen. Auch müssen keine Änderungen an der Injektionsvorrichtung vorgenommen werden, solange Form und Grösse der Schalenteile die Injektionsvorrichtung nicht stören. Bei der Konstruktion der Schalenteile muss also vor allem darauf geachtet werden, dass die Komplementärelemente geometrisch korrekt platziert sind und zum Gehäuse passen. Darüber hinaus ergeben sich zahlreiche Designmöglichkeiten, um die schlussendliche Aussenform der Nadelschutzabziehkappe an spezifische Bedürfnisse anzupassen.

Als Gesamtheit können die Schalenteile das Gehäuse (soweit im montierten Zustand ausserhalb der Injektionsvorrichtung) gänzlich umhüllen - so können die Schalenteile zum Beispiel als Halbschalen ausgebildet sein. Alternativ können die Schalenteile auch nur bestimmte Bereiche des Gehäuses überdecken.

Wie erwähnt ist das Zusammenspiel von den Elementen, Komplementärelementen und Blockierelementen so ausgelegt, dass nachdem alle Schalenteile am Gehäuse angebracht sind, sich die Schalenteile nicht mehr vom Gehäuse entfernen lassen, so dass die benutzende Person von der Mehrteiligkeit der Nadelschutzabziehkappe nichts bemerkt. Hierzu ist es wichtig, dass die Schalenteile fest und unbeweglich relativ zum Gehäuse am Gehäuse angebracht werden können.

In einem Aspekt der Erfindung umfassen die Komplementärelemente der Schalenteile einerseits Schnapparme und die Elemente auf der Gehäuseaussenfläche umfassen Vertiefungen, in welchen die Schnapparme verrastet werden können. Weiter umfassen die Komplementärelemente Führungsrippen und die Elemente Führungsnuten, welche in etwa senkrecht zur Längsachse verlaufen. Die Führungsnuten weisen Hinterschneidungen auf. Damit ein Schalenteil auf das Gehäuse aufgeschnappt werden kann, wird die betreffende Schale radial zum Gehäuse hin auf das Gehäuse geschoben, so dass die Führungsrippen in die dafür vorgesehenen Nuten eingefahren werden können. Der Querschnitt der Führungsrippen ist dabei so ausgestaltet, dass die Rippen in die Hinterschneidungen der Führungsnuten eingreifen. Das Schalenteil wird dabei soweit aufgeschoben, bis die Schnapparme in den Vertiefungen einrasten.

In einem Aspekt der Erfindung umfasst die Nadelschutzabziehkappe zwei Schalenteile, welche als Halbschalen ausgebildet sind, so dass nachdem das erste Schalenteil auf das Gehäuse geschoben wurde, das zweite Schalenteil auf der dem ersten Schalenteil gegenüberliegenden Seite des Gehäuses aufgeschoben wird, wobei die Führungsrippen des zweiten Schalenteils ebenfalls in Führungsnuten eingeschoben werden. Beim Einrasten der Schnapparme in die Vertiefungen blockieren die Blockierelemente des zweiten Schalenteils die Schnapparme des ersten Schalenteils und umgekehrt. Hierdurch wird ein Lösen der Schalenteile vom Gehäuse folgend verhindert. In einem Aspekt der Erfindung haben die Hinterschneidungen in den Führungsnuten des Gehäuses zur Folge, dass sich die Schalen in radiale Richtung nur minimal bewegen lassen, also im Rahmen des Spiels in der Verbindung Führungsnut/Führungsrippe. Bevorzugt handelt es sich bei der Kombination aus Führungsnuten und Führungsrippen um eine Schwalbenschwanzführung.

In einer alternativen Ausgestaltung der Erfindung können die Führungsnuten an den Schalenteilen und die Führungsrippen auf äusseren Oberflächen des Gehäuses angeordnet sein.

### Figuren

Figur 1 : Dreidimensionale oder perspektivische Ansicht des Autoinjektors 1 mit der Nadelschutzabziehkappe 100
Figur 2 : vereinfachte Explosionsdarstellung von Autoinjektor 1 mit Nadelschutzabziehkappe 100, wobei vom Autoinjektor nur das Gehäuse 2 und die Nadelschutzhülse 6 des Autoinjektors dargestellt sind (aus Gründen der Übersichtlichkeit sind die weiteren Teile des Autoinjektors nicht gezeigt).
Figur 3: Längsschnitt durch den Autoinjektor 1 und die Nadelschutzabziehkappe 100, wobei in dieser Darstellung auch die Spritze 3 mit Nadelschutz 4 und dem Spritzenhalter 5 dargerstellt sind.
Figur 4a : Dreidimensionale oder perspektivische Ansicht einer alternativen Ausgestaltung eines Schalenteils 120'.
Figur 4b : Dreidimensionale oder perspektivische Ansicht eines alternativen Schalenteils 120' aufgesetzt auf das Gehäuse 110.
Figur 5a : Dreidimensionale oder perspektivische Ansicht einer weiteren alternativen Ausgestaltung eines Schalenteils 120", wobei das Schalenteil hier geschnitten ist.
Figur 5b: Dreidimensionale oder perspektivische Ansicht mit Teilschnitt des weiteren alternativen Schalenteils 120" aufgesetzt auf das Gehäuse 110. Das Schalenteil ist geschnitten dargestellt.

Im Folgenden werden bevorzugte Formen der Nadelschutzabziehkappe für Injektionsvorrichtungen beschrieben, für welche die Erfindung Anwendung finden könnte, insbesondere für Autoinjektoren. Diese Beschreibung soll keinesfalls einschränkend auszulegen sein, sondern lediglich mögliche Ausgestaltungen aufzeigen. So könnte die Erfindung auch Anwendung an anderen Injektionsvorrichtungen finden, wie zum Beispiel dem Servo-Pen der Firma Ypsomed oder dem FlexPen der Firma Novo Nordisk.

Die Mechanik des Autoinjektors 1 wurde in den Figuren aus Gründen der Übersichtlichkeit weggelassen. Dem Fachmann sind gängige Mechaniken bekannt, zum Beispiel aus den Anmeldungen WO2014/146209A1 oder PCT/CH2016/000084.

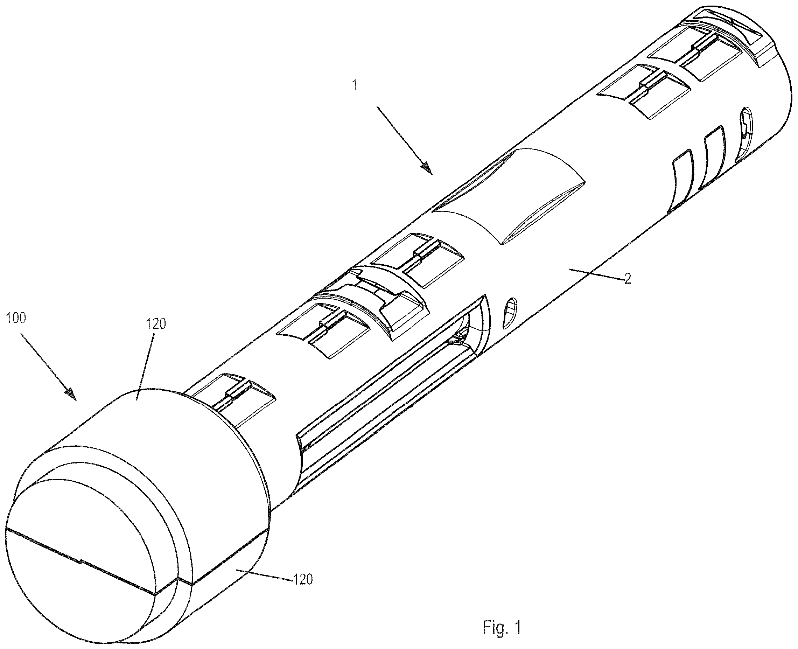

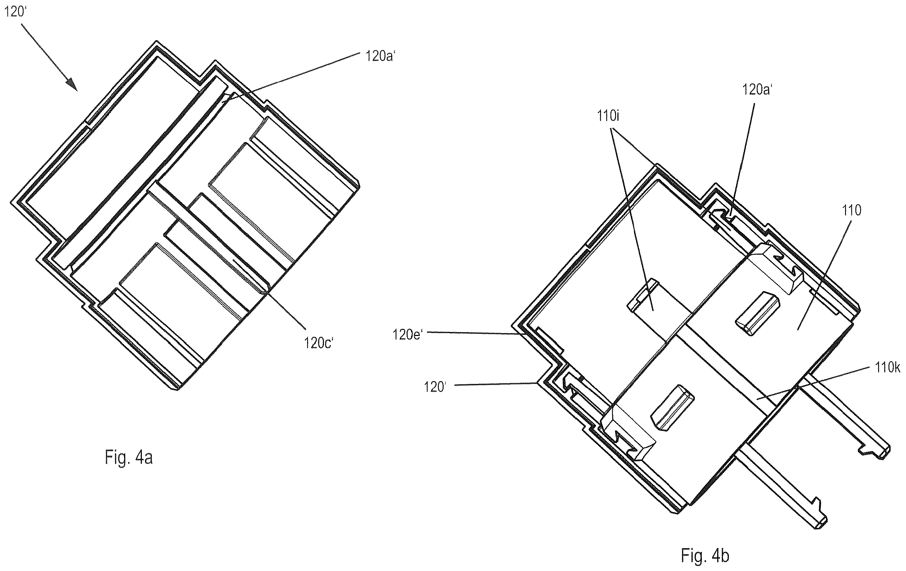

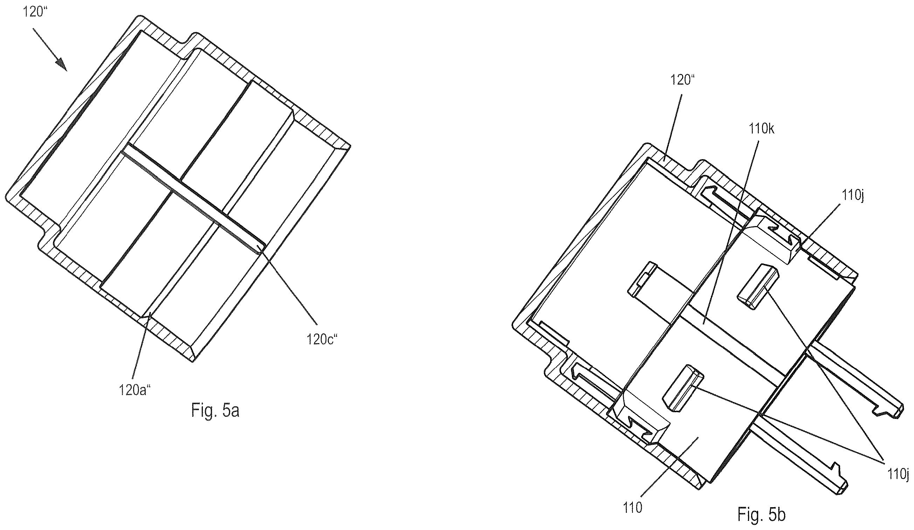

Figuren 1 bis 3 zeigen eine erste Ausführungsform der Erfindung, Figuren 4a und 4b zeigen eine alternative Ausgestaltung der Schalenteile und Figuren 5a und 5b zeigen eine vereinfachte Form mit nur einem Schalenteil.

Figur 1 zeigt den Autoinjektor 1 zusammen mit der aufgesetzten Nadelschutzabziehkappe 100 in einer dreidimensionalen Darstellung. Dabei sind zwei Schalenteile 120 zu sehen, welche das Gehäuse 110 (nicht sichtbar in Figur 1) der Nadelschutzabziehkappe 100 umgeben. Das Gehäuse 110 ist für die benutzende Person in diesem Zustand nicht sichtbar. Die äussere Form der Schalen kann wie erwähnt variieren, es ist sogar der Zweck der Erfindung, eine Variation der Aussenform (also der geometrischen Aussengestaltung der Nadelschutzabziehkappe, spezifisch der Schalenteile) zu ermöglichen. Die hier gezeigten Schalenteile sind lediglich ein Beispiel.

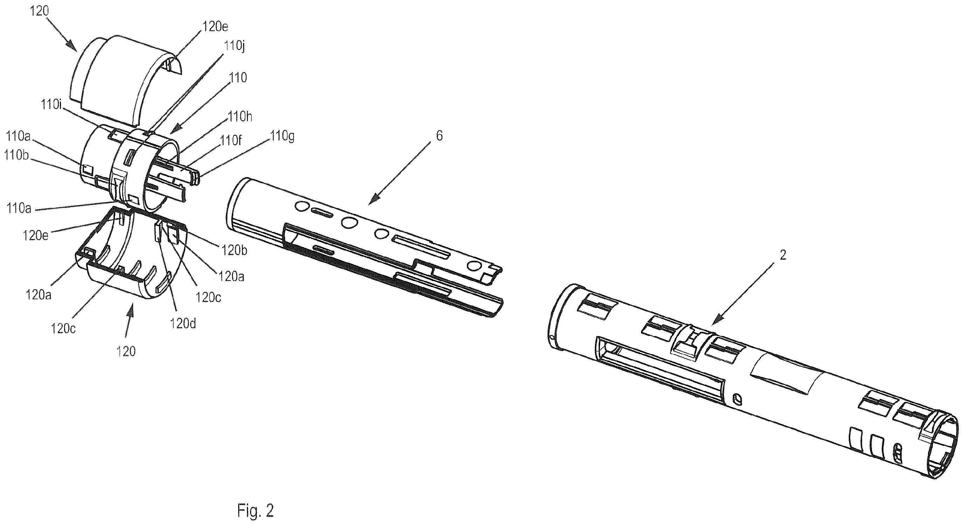

Figur 2 zeigt eine Explosionsdarstellung der Nadelschutzabziehkappe zusammen mit dem Gehäuse 2 und der Nadelschutzhülse 6 des Autoinjektors. Die weiteren Teile des Autoinjektors wurden wie oben geschrieben weggelassen. Das Gehäuse 110 umfasst die Vertiefungen 110a in die Zähne 120b der Schnapparme 120a eingeschnappt werden können. Weiter umfasst das Gehäuse 110 auch die Führungsnuten 110b, in welche die Führungsrippen 120c beim Aufschieben eines Schalenteils eingeschoben werden. Weiter umfasst ein Schalenteil auch die Blockierelemente 120e. Wie erwähnt werden bei der Montage der Nadelschutzabziehkappe 100 die Schalenteile 120 einzeln auf dem Gehäuse 110 angebracht, indem sie radial auf das Gehäuse aufgeschoben werden, wobei die Führungsrippen 120c in die Führungsnuten 110b eingeschoben werden. Die Schalenteile sind dann fertig aufgeschoben, wenn die Zähne 120b in den dafür vorgesehenen Vertiefungen 110a einschnappen. Nach dem ein erstes Schalenteil 120 montiert ist, wird ein zweites Schalenteil 120 auf der Gegenseite des Gehäuses aufgeschoben, analog zum ersten. Mit dem Aufschnappen des zweiten Schalenteils auf dem Gehäuses geraten auch die Blockierelemente 120e des einen Schalenteils 120 in Eingriff mit der Rückseite des freien Endes des Schnapparmes 120a im Bereich des Zahns 120b des anderen Schalenteils 120 und umgekehrt. Die Blockierelemente 120e verhindern ein Auslenken der Schnapparme 120a radial nach aussen (nicht gezeigt in den Figuren), wodurch ein Lösen der Schnappverbindung verhindert wird.

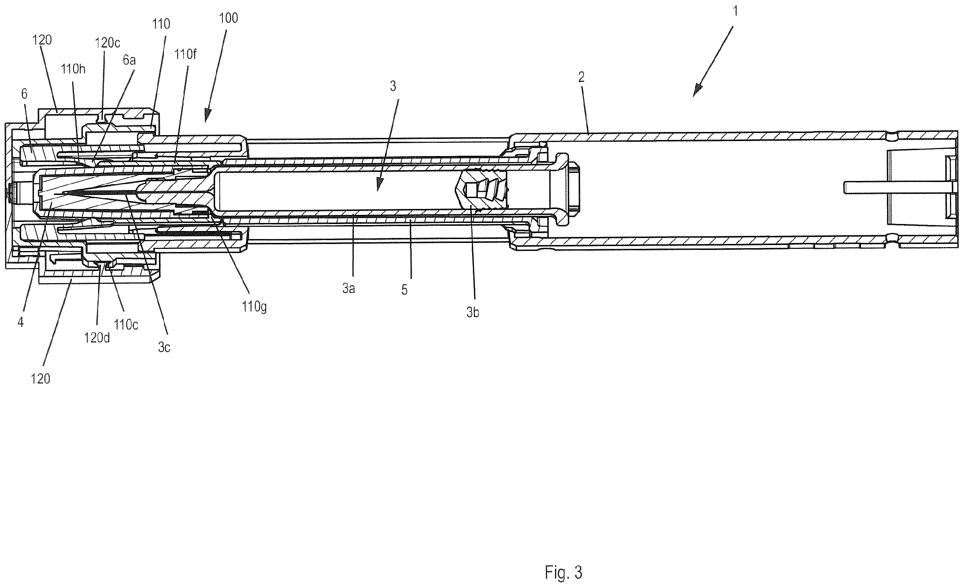

Figur 3 zeigt einen Längsschnitt durch den Autoinjektor 1 und die Nadelschutzabziehkappe 100. Gezeigt werden in dieser Darstellung auch die Spritze 3 mit Glaskörper 3a, beweglichem Stopfen 3b und der Injektionsnadel 3c, der Nadelschutz 4 sowie der Spritzenhalter 5 des Autoinjektors 1. Auch zu sehen ist der vordere Teil der Nadelschutzhülse 6 des Autoinjektor 1 mit dem Schnapparm 6a. Bei der Nadelschutzabziehkappe 100 sind die beiden Schalenteile 120 montiert. In dieser Darstellung ist erkennbar, dass die Führungsnuten 110b Hinterschneidungen 110c aufweisen. Komplementär dazu weisen die Führungsrippen 120c einen Schwalbenschwanz 120d auf, welcher in die Hinterschneidungen eingreift. Diese Ausgestaltung hat die Funktion, eine radiale Beweglichkeit der Schalenteile 120 im montierten Zustand zu minimieren und somit der Kombination aus Gehäuse 110 und Schalenteilen 120 maximale Stabilität zu verleihen. Die benutzende Person soll grundsätzlich nicht spüren, dass die Nadelschutzabziehkappe 100 mehrteilig ausgebildet ist. Die Nadelschutzabziehkappe 100 ist in axiale Richtung in den Autoinjektor 1 eingeschoben. Dabei sorgt die Verbindung aus Schnapparm 6a der Nadelschutzhülse 6 des Autoinjektors 1 und Öffnung 110h des Gehäuses 110 dafür, dass die Nadelschutzabziehkappe 100 am Autoinjektor hält, wobei diese Verbindung lösbar ist. Am Gehäuse 110 ragen zwei Arme 110f axial in proximale Richtung. An ihren freien Enden sind Zähne 110g angeordnet, welche radial nach innen schauen. Wie in Figur 3 zu sehen ist, liegen die Zähne 110g hinter dem proximalen Ende des Nadelschutzes 4. Wird nun die Nadelschutzabziehkappe 100 in distale Richtung vom Autoinjektor 1 abgezogen, so kommen das proximale Ende des Nadelschutzes 4 und die Zähne 110g in Eingriff. Bei einer weiteren Bewegung der Nadelschutzabziehkappe 100 in distale Richtung nehmen die Zähne 110g den Nadelschutz 4 mit und lösen ihn von der Spritze 3 ab, wodurch die Injektionsnadel freigelegt wird.

Figuren 4a und 4b zeigen die alternativ ausgestaltete Schalenteile 120'. Die Konstruktion der Schalenteile wurde dabei vereinfacht. Wie bei den Schalenteilen aus den Figuren 1 bis 3, werden zwei Schalenteile auf das Gehäuse 110 (welches identisch ist zu demjenigen aus den Figuren 1 bis 3) aufgebracht. Vor dem Aufschieben der Schalenteile werden zwei Schalenteile 120' entlang der rippenförmigen Vorbereitung der Schweissnaht zusammengeschweisst und dann in axiale Richtung auf das Gehäuse 110 aufgeschoben bis die Schnapparme 110i mit der Schnappkante 120a' verschnappen. Dabei wird beim Aufschieben der verschweissten Schalenteile 120' die Führungsrippen 120c' in die dafür vorgesehene Führungsnuten 110k eingeschoben. Wie im Beispiel aus den Figuren 1 bis 3 weisen auch die Führungsnuten 110k Hinterschneidungen (nicht gezeigt) und die Führungsrippen 120c' Schwalbenschwänze (nicht gezeigt) auf, so dass nach der Montage der Schalenteile 120' auf dem Gehäuse 110 eine Bewegung der Schalenteile 120' in radiale Richtung relativ zum Gehäuse 110 minimiert werden kann. Auch bei dieser Ausgestaltung stellt die äussere Geometrie der Schalen lediglich ein Beispiel dar, da es ja ein Ziel der Erfindung ist verschiedene äussere Formen zuzulassen.

Figuren 5a und 5b zeigen eine weitere Vereinfachung der Schalenkonstruktion, indem aus zwei Schalenteilen eines gemacht wurde. Beim Schalenteil 120" ist topfförmig. Anstatt zwei Schalenteilen wird bei dieser Ausgestaltung nur eine Schale gebraucht, welche in axiale Richtung auf das Gehäuse 110 aufgeschoben wird, wobei das Gehäuse 110 identisch ist zu den vorherigen Ausgestaltungen. Die Führungsrippen 120c" sowie die Führungsnuten 110k funktionieren gleich wie im Beispiel aus den Figuren 4a und 4b. Verschnappt wird das Schalenteil 120" über die Schnappkante 120a" des Schalenteiles 120" und die Schnappzähne 110j des Gehäuses 110.

Vorteilhaft an der beschriebenen Erfindung ist auch die Konstruktion des Gehäuses 110. Wie dem Fachmann aus der obenstehenden Beschreibung klar wird, erlaubt die beschriebene Konstruktion von Gehäuse 110 das Anbringen einer Vielzahl verschiedener Schalenarten, dabei muss die Konstruktion des Gehäuses 110 nicht geändert werden.

### Bezugszeichenliste:

- 1: Autoinjektor
- 2: Gehäuse des Autoinjektors
- 3: Spritze
- 3a: Glaskörper
- 3b: Stopfen
- 3c: Injektionsnadel oder Kanüle
- 4: Nadelschutz
- 5: Spritzenhalter
- 6: Nadelschutzhülse des Autoinjektors
- 6a: Schnapparm
- 100: Nadelschutzabziehkappe
- 110: Gehäuse
- 110a: Vertiefung
- 110b: Führungsnut
- 110c: Hinterschneidung
- 110d: Zahn
- 110f: Arm des Nadelschutzabzugs
- 110g: Zahn
- 110h: Öffnung
- 110i: Schnapparm mit Zahn
- 110j: axial gerichteter Schnappzahn
- 110k: Führungsnut mit Hinterschneidung
- 120: Schalenteil
- 120a: Schnapparm
- 120b: Zahn
- 120c: Führungrippe
- 120d: Schwalbenschwanz
- 120e: Blockierelement
- 120': alternative Ausgestaltung des Schalenteils
- 120a': Schnappkante
- 120e': rippenförmige Vorbereitung für Schweissnaht
- 120c': Führungsrippe mit Schwalbenschwanz
- 120": weitere alternative Ausgestaltung des Schalenteils
- 120a": Schnappkante
- 120c": Führungsrippe mit Schwalbenschwanz

## Patentansprüche

1. Nadelschutzabziehkappe (100) für ein Injektionsgerät (1) umfassend
ein Gehäuse (110), welches lösbar mit dem Injektionsgerät (1) an dessen distalen Ende verbindbar ist und zusammen mit dem Injektionsgerät (1) eine Längsachse sowie radiale Richtungen definiert,
mindestens zwei Schalenteile (120), welche auf einer äusseren Oberfläche des Gehäuses (110) anbringbar sind,
wobei auf der äusseren Oberfläche des Gehäuses (110) mehrere Haltepunkte in Form einer Vertiefung (110a) und mehrere Führungselemente (110b) angeordnet sind,
wobei die Führungselemente (110b) als längliche Nuten oder Rippen ausgebildet und in etwa senkrecht zur Längsachse stehen,
wobei auf den Innenseiten der mindestens zwei Schalenteile (120) jeweils mindestens ein Befestigungselement (120a), ein Blockierelement (120e) sowie mindestens ein Führungsgegenelement (120c) angeordnet sind,
**dadurch gekennzeichnet, dass**
die Schalenteile (120) durch Eingriff der Befestigungselemente (120a) in die Haltepunkte des Gehäuses (110a) am Gehäuse (110) angebracht werden können und das Lösen der Befestigungselemente (120a) eines Schalenteils (120) aus dem Eingriff mit einem Haltepunkt durch ein Blockierelement (120e) eines anderen, ebenfalls am Gehäuse (110) angebrachten Schalenteils (120) verhinderbar ist, so dass, wenn alle der mindestens zwei Schalenteile am Gehäuse fixiert sind, ein zerstörungsfreies Lösen der Schalenteile verunmöglicht wird, und dass die Führungsgegenelemente (120c) zu den Führungselementen (110b) komplementär als Rippen (120c) oder Nuten ausgebildet sind, und die Führungsgegenelemente (120e) beim Anbringen der Schalenteile (120) am Gehäuse in die Führungselemente (110b) eingeschoben werden,
wobei die Nuten Hinterschneidungen (110c) aufweisen und die Rippen (120c) beim Einschieben in diese Hinterschneidungen (110c) eingreifen, so dass wenn die Schalen am Gehäuse (110) angebracht sind, die Schalen in radiale Richtung relativ zum Gehäuse (110) gehalten werden.

2. Nadelschutzabziehkappe (100) nach Anspruch 1, wobei die Nadelschutzabziehkappe (100) genau 2 Schalenteile (120) umfasst.

3. Nadelschutzabziehkappe (100) nach Anspruch 1 oder 2, **dadurch gekennzeichnet, dass** sich zwischen den Nuten und Rippen (120c) beim Einschieben der Schalenteile (120) eine Schwalbenschwanzführung (110c/120d) ergibt.

4. Nadelschutzabziehkappe (100) nach einem der vorhergehenden Ansprüche, wobei die mindestens zwei Schalenteile (120) identisch sind.

5. Injektionsgerät (1) mit einer Nadelschutzabziehkappe (100) nach einem der vorhergehenden Ansprüche, wobei das Injektionsgerät (1) eine vorbefüllte Spritze (3) umfasst und es sich beim Injektionsgerät (1) um einen Injektionspen, einen Autopen, einen Autoinjektor (1) oder einer Sicherheitspritze handelt.

6. Verfahren zum Erstellen einer Nadelschutzabziehkappe nach einem der Ansprüche 1 bis 4 (100) für ein Injektionsgerät (1) enthaltend die folgenden Schritte:
- Bereitstellen eines Gehäuses (110),
- Aufschieben und Befestigen eines ersten Schalenteiles (120) auf eine Aussenfläche des Gehäuses (110),
- optionales Aufschieben und Befestigen von weiteren Schalenteilen (120) auf der Aussenfläche des Gehäuses (110),
- unlösbares Sichern aller aufgeschobenen Schalenteile (120) durch Aufschieben und Befestigen eines zweiten oder letzten Schalenteiles (120) auf das Gehäuse (110).

## Claims

1. Detachable needle protection cap (100) for an injection device (1), comprising
a housing (110), which can be detachably connected to the injection device (1) at its distal end and, together with the injection device (1), defines a longitudinal axis and radial directions,
at least two shell parts (120), which can be attached to an outer surface of the housing (110),
a plurality of holding points in the form of a depression (110a) and a plurality of guide elements (110b) being arranged on the outer surface of the housing (110),
the guide members (110b) being designed as elongate grooves or ribs and being approximately perpendicular to the longitudinal axis,
at least one fastening element (120a), a blocking element (120e) and at least one counter guide element (120c) being arranged on each of the inner sides of the at least two shell parts (120),
**characterized in that**
the shell parts (120) can be attached to the housing (110) by engagement of the fastening elements (120a) in the holding points of the housing (110a), and release of the fastening elements (120a) of a shell part (120) from the engagement with a holding point can be prevented by a blocking element (120a) of another shell part (120) which is likewise attached to the housing (110), such that, when all of the at least two shell parts are fixed to the housing, non-destructive release of the shell parts is rendered impossible, and **in that** the guide counter elements (120c) are designed to be complementary to the guide elements (110b) as ribs (120c) or grooves, and the guide counter elements (120c) are inserted into the guide elements (110b) when the shell parts (120) are attached to the housing,
the grooves having undercuts (110c) and the ribs (120c) engaging into these undercuts (110c) during insertion, such that when the shells are attached to the housing (110), the shells are held in the radial direction relative to the housing (110).

2. Detachable needle protection cap (100) according to claim 1, wherein the detachable needle protection cap (100) comprises exactly 2 shell parts (120).

3. Detachable needle protection cap (100) according to either claim 1 or claim 2, **characterized in that** a dovetail guide (110c/120d) is produced between the grooves and ribs (120c) when the shell parts (120) are inserted.

4. Detachable needle protection cap (100) according to any of the preceding claims, wherein the at least two shell parts (120) are identical.

5. Injection device (1) comprising a detachable needle protection cap (100) according to any of the preceding claims, wherein the injection device (1) comprises a pre-filled syringe (3) and the injection device (1) is an injection pen, an autopen, an auto-injector (1) or a safety syringe.

6. Method for producing a detachable needle protection cap according to any of claims 1 to 4 (100) for an injection device (1), comprising the following steps:
- providing a housing (110),
- pushing and fastening a first shell part (120) onto an outer surface of the housing (110),
- optionally pushing and fastening further shell parts (120) onto the outer surface of the housing (110),
- non-releasably securing all the pushed-on shell parts (120) by pushing and fastening a second or last shell part (120) onto the housing (110).

## Revendications

1. Capuchon détachable de protection d'aiguille (100) pour un instrument d'injection (1) comprenant
un boîtier (110), qui peut être relié de manière amovible à l'instrument d'injection (1) au niveau de son extrémité distale et qui définit, conjointement avec l'instrument d'injection (1), un axe longitudinal ainsi que des directions radiales,
au moins deux parties de coque (120), qui peuvent être appliquées sur une surface externe du boîtier (110),
plusieurs points de retenue sous forme d'un creux (110a) et plusieurs éléments de guidage (110b) étant agencés sur la surface externe du boîtier (110),
les éléments de guidage (110b) étant conçus sous forme de rainures ou de nervures longitudinales et étant environ perpendiculaires à l'axe longitudinal,
respectivement au moins un élément de fixation (120a), un élément de blocage (120e) ainsi qu'au moins un contre-élément de guidage (120c) étant agencés sur les faces internes des au moins deux parties de coque (120),
**caractérisé en ce que**
les parties de coque (120) peuvent être appliquées, par mise en prise des éléments de fixation (120a) dans les points de retenue (110a) du boîtier, au niveau du boîtier (110) et le détachement des éléments de fixation (120a) d'une partie de coque (120) de la prise avec un point de retenue peut être empêché par un élément de blocage (120e) d'une autre partie de coque (120), également appliquée au niveau du boîtier (110), de telle sorte que lorsque toutes les au moins deux parties de coque sont fixées au niveau du boîtier, un détachement non destructif des parties de coque est rendu impossible et **en ce que** les contre-éléments de guidage (120c) sont réalisés, de manière complémentaire par rapport aux éléments de guidage (110b), sous forme de nervures (120c) ou de rainures et les contre-éléments de guidage (120c) sont insérés dans les éléments de guidage (110b) lors de l'application des parties de coque (120) au niveau du boîtier,
les rainures présentant des contre-dépouilles (110c) et les nervures (120c) venant en prise dans ces contre-dépouilles (110c) lors de l'insertion, de telle sorte que lorsque les coques sont appliquées au niveau du boîtier (110), les coques sont maintenues dans la direction radiale par rapport au boîtier (110).

2. Capuchon détachable de protection d'aiguille (100) selon la revendication 1, le capuchon détachable de protection d'aiguille (100) comprenant exactement 2 parties de coque (120).

3. Capuchon détachable de protection d'aiguille (100) selon la revendication 1 ou 2, **caractérisé en ce qu'**un guidage en queue d'aronde (110c/120d) se forme entre les rainures et les nervures (120c) lors de l'insertion des parties de coque (120).

4. Capuchon détachable de protection d'aiguille (100) selon l'une des revendications précédentes, les au moins deux parties de coque (120) étant identiques.

5. Instrument d'injection (1) comportant un capuchon détachable de protection d'aiguille (100) selon l'une des revendications précédentes, l'instrument d'injection (1) comprenant une seringue préremplie (3) et l'instrument d'injection (1) étant un stylo injecteur, un Autopen, un auto-injecteur (1) ou une seringue de sécurité.

6. Procédé destiné à réaliser un capuchon détachable de protection d'aiguille (100) selon l'une des revendications 1 à 4 pour un instrument d'injection (1) contenant les étapes suivantes de :
- mise à disposition d'un boîtier (110),
- mise en place et fixation d'une première partie de coque (120) sur une surface externe du boîtier (110),
- mise en place et fixation éventuelles d'autres parties de coque (120) sur la surface externe du boîtier (110),
- sécurisation inamovible de toutes les parties de coque (120) mises en place par mise en place et fixation d'une deuxième ou dernière partie de coque (120) sur le boîtier (110).
